# EUROPEAN PATENT APPLICATION

(11) **EP 4 830 580 A1**
(43) Date of publication of application: **28.10.2026**
(21) Application number: 25172178.3
(22) Date of filing: 24.04.2025
(51) Int. Cl.: A46B 15/00, A61B 5/08, A61B 5/087, A61C 17/16

(54) **ORAL HEALTH CARE DEVICE**

(71) Applicant: Koninklijke Philips N.V., 5656 AE Eindhoven (NL)
(72) Inventor: HOETZL, Sandra, Eindhoven (NL); KOOIJMAN, Gerben, Eindhoven (NL); BALTAZAR DOS SANTOS, Nuno Filipe, Eindhoven (NL)
(74) Representative: Philips Intellectual Property & Standards

(57) **Abstract**

Proposed concepts aim to provide schemes, solutions, concepts, designs, methods, and systems pertaining to an oral health care device comprising a sensor configured such that gas exhaled by the subject is incident on the sensor for at least a portion of a time period in which an oral health care treatment is carried out on the subject. In particular, the sensor is configured to measure a gas sensor value which indicates a breathing parameter of the subject.

## Description

### FIELD OF THE INVENTION

The invention relates to oral health care devices and more particularly to oral health care devices for performing oral health care treatments.

### BACKGROUND OF THE INVENTION

Oral health care devices may be equipped to perform various oral health care treatments on a subject. For example, toothbrushes permit the teeth and gums to be cleaned through mechanical brushing action and oral irrigators provide oral cleaning via the action of fluid jets. Such devices are often used regularly by a subject (for example twice daily) to maintain good oral health.

Modern oral health care devices may be incorporated into systemic health monitoring systems that identify connections between oral health and overall health. Since oral health care treatments are often carried out frequently and consistently, such systems allow for regular non-invasive monitoring that enables the detection of early signs of systemic diseases. Links have been shown between oral health and the occurrence of conditions like diabetes, respiratory diseases, and infections.

Often oral-systemic health monitoring is achieved via the acquisition of saliva samples. However, this configuration has the significant drawback that the sensor and sampling mechanism must be placed in the portion of the oral health care device that enters the subject's mouth. This portion of the oral health care device is often a disposable component that requires replacement long before the lifetime of the sensor has expired. Further, sensors for measuring the properties of sampled saliva can often be contaminated by the presence of toothpaste or other cleaning substances in the mouth of the user and therefore can lead to unreliable measurements.

### SUMMARY OF THE INVENTION

The invention is defined by the claims.

According to examples in accordance with an aspect of the invention, there is provided an oral health care device comprising: a body adapted to be handled by a user to position the oral health care device for performing an oral health care treatment on a subject in a first time period, the body comprising: a treatment component for performing the oral health care treatment on the subject in the first time period; and a sensor configured such that, for at least a portion of the first time period, gas exhaled by the subject is incident on the sensor, and wherein, in use, the sensor is adapted to measure a gas sensor value describing a parameter of the gas exhaled by the subject, the gas sensor value being indicative of a breathing parameter of the subject.

Proposed concepts thus aim to provide schemes, solutions, concepts, designs, methods, and systems pertaining to an oral health care device comprising a sensor configured such that gas exhaled by the subject is incident on the sensor for at least a portion of a time period in which an oral health care treatment is carried out on the subject. In particular, the sensor is configured to measure a gas sensor value which indicates a breathing parameter of the subject.

One aspect of subject health that is of particular interest to measure through oral-systemic health monitoring is subject stress. Conventionally, this may be done via the measurement of cortisol levels in saliva. Cortisol is a steroid hormone produced by the adrenal glands in response to stress. It's measurable and predictable response to stressors means that it can provide critical insights in the body's stress response. However, the integration of sensors into oral health care devices for the measurement of salivary cortisol has several drawbacks. The sensors must be located on the portion of the oral health care device that is placed in the oral cavity and often this is a disposable component needing frequent replacement. Further, such sensors may suffer from contamination from other substances in the oral cavity of the subject such as toothpaste, and the measurement of saliva may have too many confounding elements for cortisol to be targeted independently.

It was realised that a suitable alternative to cortisol measuring for monitoring subject stress, involves subject breathing monitoring. Stress measurement through breathing patterns is an advanced approach to determining an individual's mental and physiological status. When under stress, breathing typically becomes rapid and shallow, altering the levels of oxygen and carbon dioxide in the blood. Breathing parameters such as breathing depth and rate variability are therefore important indicators of stress.

The present invention leverages the measurement of gas exhaled by the user to provide an oral health care device suitable for systemic health monitoring including breathing and stress monitoring. In particular, the oral health care device of the present invention is equipped with a sensor capable of analysing the gas exhaled by the user during the performance of an oral health care treatment so as to measure a gas sensor value that indicates a breathing parameter of the subject.

Ultimately, an improved oral health care device suitable for systemic health monitoring may be provided by the proposed concept(s).

In some embodiments, the sensor may comprise at least one of: a flow sensor; an oxygen sensor; a carbon dioxide sensor; a gas compound concentration sensor; a VOC sensor; a pressure sensor; and a differential pressure sensor. Each of these sensors may be suitable for measuring an aspect of the gas incident on the sensor and enable information about the breathing of the subject to be derived.

In some embodiments, the gas sensor value may comprise at least one of: a gas flow rate; a gas compound concentration; a pressure value; a VOC concentration; an oxygen concentration; and a carbon dioxide concentration. Each of these values may be used to derive accurate and valuable information about the subject's breathing pattern.

In some embodiments, the treatment component may be detachable from the body of the oral health care device such that the treatment component comprises a replaceable treatment component. The concepts of the present invention may be particularly advantageous in oral health care devices that have replaceable treatment components as it may be difficult to implement saliva sampling in such devices.

In some embodiments, the breathing parameter may comprise at least one of: a breathing frequency; a deviation in breathing frequency; a breathing pattern; a breathing depth; a duration of an exhalation phase; a duration of an inhalation phase; a VOC concentration of air exhaled by the subject; an oxygen concentration of air exhaled by the subject; and a carbon dioxide concentration of air exhaled by the subject. Each of these breathing parameters may provide valuable insights into subject's breathing pattern and can be used to derive information about the subject's wider physiological and psychological health.

In some embodiments, the oral health care device may further comprise an inertial measurement unit (IMU) configured to determine the orientation of the oral health care device when in use. In certain embodiments of the invention, gas exhaled by the subject may only be incident on the sensor when the oral health care device is in a certain orientation with respect to the subject's nose and mouth. As such, a device fitted with an IMU may advantageously be able to sense when the device is in the correct orientation for breathing monitoring to take place and only measure the gas sensor value when the device is in this orientation.

In some embodiments, the sensor may be located within a cavity of the body, and the body may further comprise a permeable membrane at the entrance to the cavity. This may protect the sensor from damage during handling of the device whilst still allowing for gases exhaled by the subject to be incident on the sensor.

In some embodiments, the body may further comprise a handle portion configured to be held by the subject during use of the oral health care device. Hence, the concepts of the present invention may be advantageously applied to personal handheld oral health care devices to allow for non-invasive regular monitoring of subject systemic health.

In some embodiments, the body may comprise a housing configured to support at least one of: the treatment component; and the sensor. The housing may also be used to house other optional components of the oral health care device

According to another aspect of the invention, there is provided subject health monitoring system comprising: any one of the oral health care devices described above; a data acquisition module configured to acquire the gas sensor value from the oral health care device; and a data analysis module configured to determine, based on the measured gas sensor value, a value of the breathing parameter of the subject. Thus, the oral health care device of the present invention may be incorporated into a system that enables the breathing of the subject to be analysed.

In some embodiments, the data analysis module is further configured to determine a stress metric describing a predicted stress level of the subject based on the determined value of the breathing parameter. Hence the system may be adapted to allow for the stress of the subject to be reliably monitored during the frequent performance of the oral health care treatment by the subject.

In some embodiments, the oral health care device may further comprise an inertial measurement unit (IMU) configured to determine the orientation of the oral health care device, and determining the value of the breathing parameter of the subject may be further based on the determined orientation of the oral health care device. This advantageously enables the breathing parameter to only be determined when the oral health care device is in the correct orientation to suggest that gas incident on the sensor is gas exhaled by the subject.

According to a third aspect of the invention, there is provided a method for monitoring the health of a subject, the method comprising: performing, using any of the oral health care devices described above, an oral health care treatment on the subject in a first time period; measuring, by the sensor of the oral health care device, a gas sensor value describing a parameter of gas incident on the sensor in at least a portion of the first time period, wherein the gas sensor value is indicative of a breathing parameter of the subject; and determining, based on the measured sensor value, a value of the breathing parameter of the subject.

In some embodiments, the method may further comprise determining a stress metric describing a predicted stress level of the subject based on the determined value of the breathing parameter. The present invention therefore advantageously permits non-invasive assessment of subject stress levels.

Embodiments may be employed in combination with conventional/existing oral health care devices. In this way, embodiments may integrate into legacy systems to improve and/or extend their functionality and capabilities.

These and other aspects of the invention will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified diagram of an oral health care device according to an embodiment of the invention;
Figs. 2A and 2B depict an oral health care device, according to an embodiment of the invention, when it is being used by a subject;
Fig. 3 is a graph depicting the variation in the measured carbon dioxide concentration during a breathing cycle of the subject;
Fig. 4 is a simplified block diagram of a subject health monitoring system according to another aspect of the invention; and
Fig. 5 is a simplified flow diagram of a method for monitoring the health of a subject according to a third aspect of the invention.

### DETAILED DESCRIPTION OF EMBODIMENTS

The invention will be described with reference to the Figures.

It should be understood that the detailed description and specific examples, while indicating exemplary embodiments of the apparatus, systems, and methods, are intended for the purpose of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes, and/or solutions pertaining to oral health care devices. According to proposed concepts, a number of possible solutions may be implemented separately or jointly. That is, although these possible solutions may be described below separately, two or more of these possible solutions may be implemented in one combination or another.

Oral-systemic health monitoring involves monitoring the overall health of a subject through the performance of oral health care treatments. Since most of the population regularly and consistently perform oral health care treatments (e.g., twice daily brushing of teeth), combined oral and systemic health monitoring can provide a reliable non-intrusive and non-invasive way of monitoring an individual's health.

Stress is an important aspect of overall health and can have an impact on a wide variety of physiological processes including cognitive performance and thyroid function. One way by which regular stress monitoring might be achieved is via the measurement of salivary cortisol levels during oral health care treatments - cortisol is an important biomarker for stress. However, integration of a suitable sensor for measuring salivary cortisol into an oral health care device is challenging. The present invention provides an alternative method to salivary cortisol monitoring by which the stress of a subject may be monitored and assessed during the performance of an oral health care treatment. In the proposed devices, systems and methods, a sensor is integrated into an oral health care device and positioned such that, for at least a portion of an oral care session in which an oral health care treatment is performed on a subject, gas exhaled by the subject is incident on the sensor and can be measured. This solution does not suffer from the same drawbacks as salivary cortisol measurements since the sensor is not restricted to being placed in the portion of the device which enters the subject's mouth.

Measurement of the gas exhaled by the subject may provide crucial insights into the subject's breathing patterns and behaviour. These insights may then be used to derive information about the subject's health, and in particular about the level of stress of the subject. When under stress, breathing typically becomes rapid and shallow, altering levels of oxygen and carbon dioxide in the blood, potentially leading to hypoxia (low oxygen) and hypocapnia (low carbon dioxide). This alteration occurs because rapid, shallow breathing reduces oxygen intake and increases carbon elimination. Therefore, disruptions to the relative concentrations of various gas compounds (in particular oxygen and carbon dioxide) in exhaled gas can indicate heightened stress and are key markers for analysing the stress state of a subject. Other factors that might indicate stress levels are breathing depth and rate variability, since in a stressed state breathing becomes more erratic and less consistent. Additionally, the amplitude and flow intensity of breathing may increase as the subject's stress level increases.

The breathing pattern of a patient may also indicate other wider health care issues, beyond the stress state of the subject. For example, certain variations and changes in breathing patterns and behaviour may be indicative of the early signs of certain respiratory diseases.

In the present invention a suitable sensor is integrated into an oral health care device to measure the gases exhaled by the subject during at least a portion of the performance of an oral health care treatment. This provides a simple way of reliably and consistently measuring subject breathing to infer information about the subject's stress levels and wider overall health.

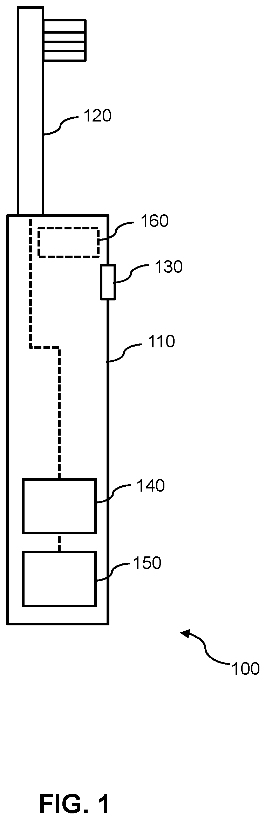

Referring now to Fig. 1, there is depicted a simplified diagram of an oral health care device 100 according to a proposed embodiment of the invention.

The oral health care device 100 comprises a body 110 adapted to be handled by a user to position the oral health care device for performing an oral health care treatment on a subject in a first time period. In this particular embodiment, the body 110 comprises a handle portion configured to be held by the subject during use of the oral health care device 100, and as such the oral health care device 100 is a personal handheld oral health care device. The subject may manipulate the handle portion of the oral health care device in order to perform the oral health care treatment.

The body 110 further comprises a treatment component 120 for performing the oral health care treatment on the subject in the first time period. In this particular embodiment, the treatment component is a brushing component which, in use, engages with the teeth and gums of the user to perform a cleaning treatment. The brushing component 120 may be detachable from the handle portion of the body 110 of the oral health care device such that the treatment component 120 comprises a replaceable treatment component, or alternatively may be permanently fixed to the body 110. In this exemplary embodiment, the oral health care device 100 is an electrically powered toothbrush and therefore further comprises a motor 140 and a battery 150 for driving vibrations of the treatment component to achieve improved cleaning performance.

The body 110 of the oral health care device 100 further comprises a sensor 130 configured such that, for at least a portion of the first time period in which the oral health care treatment is performed on the subject, gas exhaled by the subject is incident on the sensor. This result can be achieved by the correct positioning of the sensor 130 on or within the body of the oral health care device. During the oral health care treatment, the oral health care device 100 is manipulated into multiple different orientations to ensure comprehensive cleaning of the subject's oral surfaces. It is sufficient for the present invention that in at least one of these orientations, gas exhaled by the user is incident on the sensor. This allows for exhaled gas to be measured by the sensor 130 during at least a portion of the first time period in which the cleaning treatment is carried out.

The sensor 130 is adapted to measure a gas sensor value describing a parameter of the gas exhaled by the subject. The measured gas sensor value is indicative of a breathing parameter of the subject. In this exemplary embodiment, the sensor is positioned on the exterior of the body 100 and comprises a flow sensor configured to measure the gas flow rate of exhaled gas incident on the sensor. The gas flow rate of exhaled gas may be indicative of the breathing depth and frequency of the subject.

Optionally, the oral health care device 100 may further comprise an inertial measurement unit (IMU) 160 configured to determine the orientation of the oral health care device when in use. In some orientations of the oral health care device 100, gas exhaled by the user is not incident on the sensor 130 and as such exhaled gas may only incident on the sensor for a portion of the use session of the device. The IMU 160 is configured to detect when the oral health care device is in the correct orientation for measuring exhaled gases and therefore inform which measurements of the sensor 130 are indicative of a breathing parameter of the subject.

In this example, the oral health care device 100 is a powered toothbrush device which the user manipulates via the handle portion of the body 110 to pass along the surfaces of their teeth and gums to carry out oral cleaning. However, in alternative embodiments this need not be the case. It will be understood by those working in the field that the features of the invention may also be applied to other types of manual and powered oral health care devices. For example, the treatment component 120 need not be a brush head but may comprise any treatment component suitable for performing an oral health care treatment on a subject - for example a mouthpiece component or an oral irrigator jet. Similarly, whilst in this embodiment, the body comprises a handle portion such that the oral health care device 100 is a handheld device, in other embodiments, the body may simply comprise a housing for supporting at least one of the treatment component 120 and the sensor 130.

The oral health care device 100 need not comprise the IMU 160. For example, in some instances it may be evident from the gas sensor values measured by the sensor 130 when the gas incident on the sensor is gas that has been exhaled by the subject (for example a peak in the carbon dioxide concentration or the flow rate may be measured). Therefore, in some embodiments, there is no need for a separate component for detecting the orientation of the oral health care device, as time intervals associated with subject exhalation can be clearly determined from the measured signal.

Whilst in the oral health care device 100 the sensor 130 is a flow sensor, the principles of the invention may be implemented using a variety of different sensor types. For example, the sensor 130 may comprise at least one of: a flow sensor; an oxygen sensor; a carbon dioxide sensor; a gas compound concentration sensor; a volatile organic compound (VOC) sensor; a pressure sensor; and a differential pressure sensor. Therefore, the sensor need not measure the flow rate of gas incident of the sensor but may instead, or additionally, measure at least one of: a gas compound concentration; a pressure value; a VOC concentration; an oxygen concentration; and a carbon dioxide concentration. Since exhaled gas has a different elemental composition to ambient air, the concentration of various compounds in the gas incident on the sensor may provide valuable information about subject breathing. Further, the gas flow rate and fluid pressure on the sensor during the exhalation of the subject will both vary with the breathing rate and breathing depth of the subject. As such, the sensor 130 of the present invention is configured to measure one or more of any of the parameters of the exhaled gas of the subject that may indicate something about the subject's breathing.

In Fig. 1 the sensor is depicted as being positioned at a particular point on an exterior surface of the handle portion of the body 110. However, the present invention is not limited to such and the only restriction on the positioning of the sensor is that for at least a portion of the first time period, in which an oral health care treatment is carried out using the device, gas exhaled by the subject is incident on the sensor. Therefore, the sensor may be positioned differently depending on the specific geometry of the oral health care device 100. For example, the sensor may be located within a cavity in the body 110 and the body may further comprise a permeable membrane at the entrance to the cavity. This provides a way for the sensor to be protected within the interior of the device whilst still allowing accurate gas sensor measurements to be made.

The present invention therefore provides a mechanism by which breathing monitoring may be incorporated into an oral health care device. Use of such devices can advantageously allow for consistent non-invasive insights into a subject's breathing which can be used to assess the overall health of the subject.

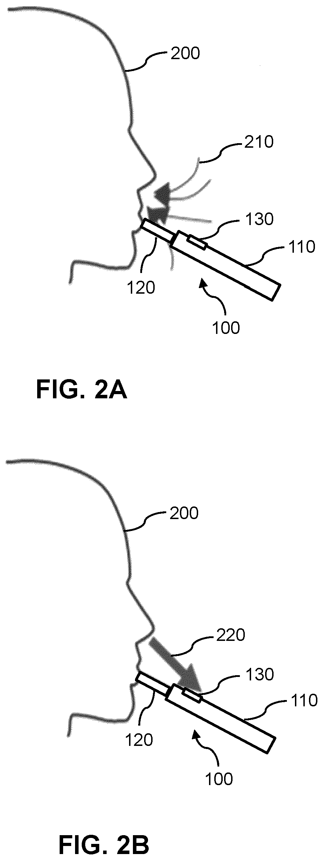

Referring now to Figs. 2A and 2B, there is depicted the oral health care device 100 when it in use by a subject 200.

During use, the oral health care device 100 is positioned such that the treatment component 120 engages with the oral surfaces of the subject 200 to perform an oral health care treatment. Figs. 2A and 2B show a particular orientation of the oral health care device 100 in which gas exhaled by the subject to be incident on the sensor 130. In this position, gas exhaled by the subject may be incident on the sensor 130 both in the case where the subject breathes through their mouth and in the case where the subject breathes through their nose.

Fig. 2A depicts the flow 210 of gas during an inhalation phase of the subject. In this phase, the subject 200 draws air from all directions as indicated by the arrows 210. Due to the multidirectional nature of this flow, virtually no flow is incident on the sensor 130 during the inhalation phase.

Fig. 2B depicts the flow 220 of gas during an exhalation phase of the subject. In this phase, the subject expels exhalation air in the form of a jet. In this particular orientation of the oral health care device 100, the jet is directed towards the handle portion of the oral health care device 100. As such, during exhalation the flow of gas expelled by the subject is incident on the sensor 130 and measured to determine the value of the gas sensor value.

Therefore, since gases are more spatially directed towards the sensor 130 during the exhalation phase than during the inhalation phase, the sensor 130 of the oral health care device 100 may be configured to predominantly measure the gas exhaled by the subject. Hence, during the exhalation phase, the measured gas sensor value provides accurate and reliable information about a parameter of the gas exhaled by the subject which is not confounded by information about the ambient air.

The concepts of the present invention involve the measurement of one or more gas sensor values which describe a parameter of the gas exhaled by the subject during at least a portion of an oral health care device usage session. The gas sensor value is measured by a sensor 130 of an oral health care device 100. The properties of gas exhaled by the subject indicated by the gas sensor value can provide information about a breathing parameter of the subject.

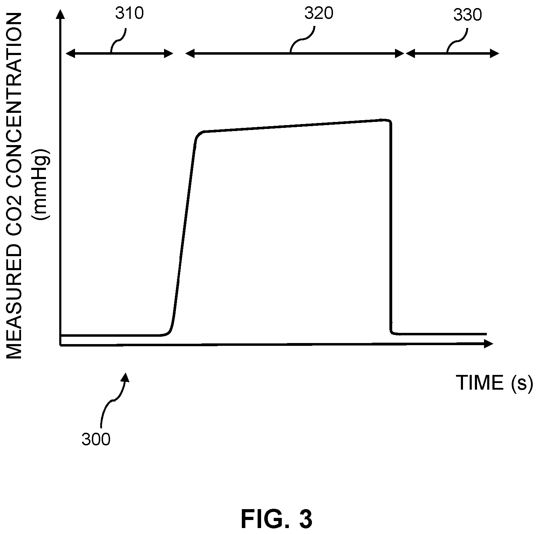

Fig. 3 depicts a graph 300 showing the variation in a carbon dioxide concentration during a breathing cycle of the subject as measured by a sensor of an oral health care device. The carbon dioxide concentration is measured by an embodiment of the sensor 130 of the oral health care device 100 and therefore, in this example, the sensor 130 comprises a carbon dioxide sensor.

In a first inhalation phase 310 the carbon dioxide concentration of the gas incident on the sensor is low. In this phase, the subject is not exhaling and so any gas incident on the sensor is ambient air. In a first exhalation phase 320, the carbon dioxide concentration measured by the sensor 130 is much higher. Exhaled gas has a much higher carbon dioxide concentration than ambient air and therefore when the gas exhaled by the subject is incident on the sensor during the exhalation phase, the CO₂ level measured by the sensor will increase. When the subject ceases to exhale and starts to inhale again, in a second inhalation phase 330, the carbon dioxide concentration on the sensor drops back to a low level as the sensor is now only exposed to ambient air rather than exhaled air.

Therefore, as can be seen in Fig.3, the variation in the carbon dioxide concentration measured by the sensor is linked to the breathing phases of the subject. Hence, various breathing parameters of the subject may be derived from the measurements made by the sensor 130. By analysing the length of time for which the carbon dioxide concentration measured by the sensor is at an elevated level or base level, the duration of the exhalation and inhalation phases respectively can be derived. When this is measured over several cycles, this information can be used to derive a breathing frequency, a deviation in the breathing frequency and a breathing pattern of the subject. The breathing depth may be determined by the total amount of carbon dioxide detected over the exhalation phase.

Thus, as illustrated above, measuring the carbon dioxide concentration of gas incident on a sensor throughout a usage session of an oral health care device provides various insights into the subjects breathing pattern. Similar principles apply to a measured signal that describes the oxygen concentration or any other gas compound concentration, and signals describing the gas flow rate or pressure. Each of these values will vary during the breathing cycle of the subject and therefore can be analysed to derive the breathing parameters of the subject.

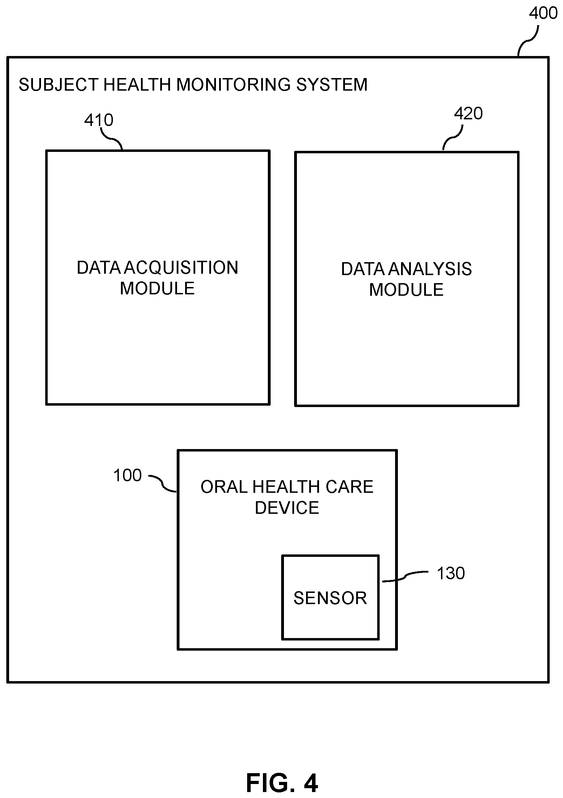

Referring now to Fig. 4, there is depicted a simplified block diagram of a subject health monitoring system 400 according to another aspect of the invention.

The subject health monitoring system comprises the oral health care device 100 including the sensor 130. Any alternative embodiments of the oral health care device 100 of the present invention may alternatively be included in the subject health monitoring system 100.

The system 400 further comprises a data acquisition module 410 configured to acquire the gas sensor value from the oral health care device 100, and a data analysis module 420 configured to determine, based on the measured gas sensor value, a value of the breathing parameter of the subject. Whilst shown as separate components in Fig. 4, in alternative embodiments, the functions of the data acquisition module 410 and the data analysis module 420 may be performed by a single processing component.

Since the sensor 130 of the oral health care device 100 is configured such that for at least a portion of the use session of the oral health care device gas exhaled by the subject is incident on the sensor, the gas sensor value measured by the sensor 130 is indicative of a breathing parameter of the subject. For example the gas sensor value may comprise information about at least one of: a breathing frequency, a deviation in breathing frequency; a breathing pattern; a breathing depth; a duration of an exhalation phase; a duration of an inhalation phase; a volatile organic compound (VOC) concentration of air exhaled by the subject; an oxygen concentration of air exhaled by the subject; and a carbon dioxide concentration of air exhaled by the subject.

The sensor may thus be configured to obtain a measurement of the gas sensor value (which may be a gas flow rate, or a gas compound concentration value for example) which can then be analysed to obtain information about the breathing pattern of the subject. Breathing frequency for example can be straightforwardly determined with either a flow sensor, an oxygen concentration sensor, or a carbon dioxide sensor by analysing the variation in the signal detected by the sensor. This may involve calculating the fundamental frequency in a Fourier transform of the sensor signal. Breathing depth may be determined using a flow (velocity) sensor by calculating the total volume of flow at the sensor over an exhalation phase of the subject. A higher measured flow volume at the sensor corresponds to a larger exhaled air volume and thus deeper breathing. Breathing depth may also be measured using gas sensors configured for measuring the concentration of a particular gas compound in the air exhaled by the subject. In particular, oxygen concentration and carbon dioxide concentration in exhaled air is directly linked to the depth of breathing.

In a particular exemplary embodiment of the invention, the data analysis module 420 is further configured to determine a stress metric describing a predicted stress level of the subject based on the determined value of the breathing parameter. Thus, the sensor 130 of the oral health care device measures a gas sensor value which can be used to derive information about the subject's breathing pattern and analysis of the breathing pattern is used in turn to inform an estimation of the stress level of the subject. In some embodiments, the stress metric may be determined based on measurements made by the sensor of the oral health care device over several usage sessions. This may provide a more reliable estimation of subject stress levels.

The depth and variability of a subject's breathing pattern may be directly correlated to the stress level of the subject. When under stress, the subject may unconsciously have a poorer breathing pattern characterised by a higher breathing frequency and a lower breathing depth. Therefore, breathing monitoring provides a non-invasive insight in the subject's stress levels.

In some embodiments, the subject health monitoring system 400 may comprise further components not depicted in Fig. 4. For example, the subject health monitoring system may comprise an output interface for displaying information to the user. The output interface may be configured to display the determined breathing parameter to the user in real time. This may allow the user to improve their breathing pattern whilst the oral health care treatment is being performed. In embodiments of the invention in which a stress metric is calculated based on the breathing parameter, this may also be displayed to the subject via the output interface.

In one exemplary embodiment of the system 400, when a poor breathing pattern is indicated by the measured values of the gas sensor value, an indication of this fact is provided to the user via the output interface. This indication could include: prompts to the subject to engage in specific breathing exercises such as 4-7-8 breathing; real-time biofeedback of the subject's breathing parameters; and/or guided meditation exercises to improve respiratory function. Additionally, or alternatively, the subject may receive, via the output interface, suggestions for physical activities such as yoga, posture corrections, and hydration reminders with the aim of improving the subject's breathing and/or stress status. In some embodiments, the system 400 is configured to output a warning to the subject when the stress metric indicates that the stress level of the subject is above a predetermined acceptable threshold level.

In embodiments where data is obtained across several usage sessions of the oral health care device 100, graphs depicting the variation in the breathing parameters and the stress metric of the user over time may be displayed to the user via a suitable output interface. Where warnings of high stress levels are given to the subject, these may be given when a sudden deterioration of the breathing pattern is observed and/or when a gradual deterioration in the breathing pattern is observed over time across several usage sessions of the oral health care device 100.

In embodiments in which the oral health care device 100 comprises an IMU, information from the IMU may be used by the system 400 in conjunction with information from the sensor 130. As such, in some embodiments, determining the value of the breathing parameter of the subject based on the gas sensor value may be further based on the orientation of the oral health care device as measured by the IMU. For example, the breathing parameter of the subject may only be determined based on gas sensor values corresponding to a time wherein the oral health care device is within a certain orientation under the nose of the subject. This may improve the consistency and robustness of the determined breathing parameter value.

Further forms of information may be used in addition to the gas sensor value to inform the determination of the breathing parameter. For example, the subject's heart rate variability (HRV) and galvanic skin response (GSR) may be measured by sensors in the handle of the oral health care device and used to improve the estimation of the stress metric. Other information that might be used include subject sleep patterns, physical activity levels, dietary habits, environmental factors, demographic variables like age and gender, and medical information such as medications being taken by the subject and known health conditions the subject has. This information may be stored within a memory component of the system 400 (not shown) or may be provided by the subject via an input interface.

The subject health monitoring system 400 therefore provides comprehensive monitoring of the subjects breathing patterns and behaviour via the oral health care device 100.

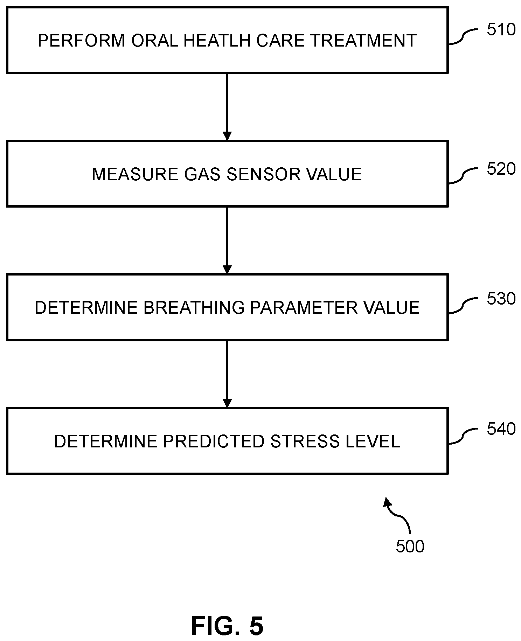

Referring now to Fig. 5, there is depicted a simplified flow diagram of a method 500 for monitoring the health of a subject.

The method 500 commences with a step 510 comprising, performing, using the oral health care device 100, an oral health care treatment on a subject.

In step 520 of the method 500, the sensor 130 of the oral health care device 100 measures a gas sensor value describing a parameter of gas incident on the sensor. The sensor 130 of the oral health care device is configured such that during at least a part of the performance of the oral health care treatment, gas exhaled by the subject is incident on the sensor. As such, the gas sensor value is indicative of a breathing parameter of the subject.

The method 500 then proceeds to step 530 which comprises determining, based on the measured gas sensor value, a value of the breathing parameter of the subject. In this exemplary embodiment of the method 100, the method further comprises a step 540 in which the determined value of the breathing parameter is used to determine a stress metric describing a predicted stress level of the subject.

The method 500 therefore allows for systemic monitoring of subject breathing and stress levels via an oral health care device equipped to measure gas exhaled by the subject.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module segment, or portion of instruction, which comprises one or more executable instructions for implementing the specified logical function(s). In some alternative implementations, the functions noted in the block may occur out of the order noted in the figures. For example, two blocks shown in succession may, in fact be executed substantially concurrently, or the blocks may sometimes be executed in reverse order, depending on the functionality involved.

## Claims

1. An oral health care device (100) comprising:
a body (110) adapted to be handled by a user to position the oral health care device for performing an oral health care treatment on a subject in a first time period, the body comprising:
a treatment component (120) for performing the oral health care treatment on the subject in the first time period; and
a sensor (130) configured such that, for at least a portion of the first time period, gas exhaled by the subject is incident on the sensor,
wherein, in use, the sensor is adapted to measure a gas sensor value describing a parameter of the gas exhaled by the subject, the gas sensor value being indicative of a breathing parameter of the subject.

2. The oral health care device of claim 1, wherein the sensor comprises at least one of:
a flow sensor;
an oxygen sensor;
a carbon dioxide sensor;
a gas compound concentration sensor;
a volatile organic compound (VOC) sensor;
a pressure sensor; and
a differential pressure sensor.

3. The oral health care device of any of claims 1 and 2, wherein the gas sensor value comprises at least one of:
a gas flow rate;
a gas compound concentration;
a pressure value;
a VOC concentration;
an oxygen concentration; and
a carbon dioxide concentration.

4. The oral health care device of any preceding claim, wherein the treatment component is
detachable from the body of the oral health care device such that the treatment component comprises a replaceable treatment component.

5. The oral health care device of any preceding claim, wherein the breathing parameter comprises at least one of:
a breathing frequency;
a deviation in breathing frequency;
a breathing pattern;
a breathing depth;
a duration of an exhalation phase;
a duration of an inhalation phase;
a VOC concentration of air exhaled by the subject;
an oxygen concentration of air exhaled by the subject; and
a carbon dioxide concentration of air exhaled by the subject.

6. The oral health care device of any preceding claim, wherein the oral health care device further comprises an inertial measurement unit (160) configured to determine the orientation of the oral health care device when in use.

7. The oral health care device of any preceding claim, wherein the sensor is located within a cavity in the body and wherein the body further comprises a permeable membrane at the entrance to the cavity.

8. The oral health care device of any preceding claim, wherein the body comprises a handle portion configured to be held by the subject during use of the oral health care device.

9. The oral health care device wherein the body comprises a housing configured to support at least one of:
the treatment component; and
the sensor.

10. A subject health monitoring system (400) comprising:
the oral health care device (100) of any of claims 1-9;
a data acquisition module (410) configured to acquire the gas sensor value from the oral health care device; and
a data analysis module (420) configured to determine, based on the measured gas sensor value, a value of the breathing parameter of the subject.

11. The subject health monitoring system of claim 10, wherein the data analysis module is further configured to determine a stress metric describing a predicted stress level of the subject based on the determined value of the breathing parameter.

12. The subject health monitoring system of any of claims 10 and 11, wherein the oral health care device further comprises an inertial measurement unit (IMU) configured to determine the orientation of the oral health care device, and
wherein determining the value of the breathing parameter of the subject is further based on the determined orientation of the oral health care device.

13. A method (500) for monitoring the health of a subject, the method comprising:
performing (510), using the oral health care device of any of claims 1-8, an oral health care treatment on a subject in a first time period;
measuring (520), by the sensor of the oral health care device, a gas sensor value describing a parameter of gas incident on the sensor in at least a portion of the first time period, wherein the gas sensor value is indicative of a breathing parameter of the subject; and
determining (530), based on the measured gas sensor value, a value of the breathing parameter of the subject.

14. The method of claim 13, wherein the method further comprises determining (540) a stress metric describing a predicted stress level of the subject based on the determined value of the breathing parameter.
